# EUROPÄISCHE PATENTSCHRIFT

(11) **EP 1 940 330 B1**
(45) Veröffentlichungstag und Bekanntmachung des Hinweises auf die Patenterteilung: **31.03.2010**
(21) Anmeldenummer: 06804368.6
(22) Anmeldetag: 27.10.2006
(51) Int. Cl.: A61F 5/03, A61F 5/30

(54) **Stützvorrichtung für den Brustkorb**
Thorax supporting device
Dispositif de support pour cage thoracique

(30) Priorität: 28.10.2005 AT 17682005
(43) Veröffentlichungstag der Anmeldung: 09.07.2008
(73) Patentinhaber: GARVINGTON RESEARCH&DEVELOPMENT INC., Clearwater, FL 33756 (US); HEARTSEASE THERAPY, LLC, Gates Mills, OH 44040 (US)
(72) Erfinder: EPPLE, Jürgen, A-1230 Wien (AT); SVENSSON, Lars, Gates Mills, OH 44040 (US)
(74) Vertreter: Haffner und Keschmann Patentanwälte OG
(86) Internationale Anmeldenummer: PCT/AT2006/000440
(87) Internationale Veröffentlichungsnummer: WO 2007/048162

(56) Entgegenhaltungen:
- FR-A- 2 670 668
- US-A- 2 800 902
- US-A- 4 022 197
- US-A- 4 682 588
- US-A- 5 011 452
- US-A- 5 551 085

## Beschreibung

Die Erfindung betrifft eine Stützvorrichtung für den Brustkorb mit wenigstens einem den Brustkorb umschließenden Zugelement und Auflageelementen, die mit Hilfe des wenigstens einen Zugelements gegen den Brustkorb andrückbar sind.

Derartige Stützvorrichtungen kommen beispielsweise als Rippenbruchgürtel zum Einsatz und dienen dazu den Brustkorb nach einem Rippenbruch zu stabilisieren, um die Verheilung zu beschleunigen. Derartige Rippenbruchgürtel bestehen beispielsweise aus einem den Brustkorb umschließenden Gurt, welcher als Zug- bzw. Spannelement wirksam wird und durch entsprechendes Festspannen eine Einschränkung der Thoraxdehnung während der Atmung bewirkt. Insbesondere nach Brustöffnungen stützt ein solcher Rippenbruchgürtel zusätzlich die Wirbelsäule und wirkt somit Rückenschmerzen entgegen.

Ein Rippenbruchgürtel ist beispielsweise aus der GB 626425 A bekannt geworden.

Aus der WO 96/28129 A1 ist eine ähnliche Vorrichtung bekannt geworden, welche jedoch nicht als Rippenbrustgürtel Verwendung findet, sondern als Herz-Lungen-Wiederbelebungsgerät. Das Gerät besteht aus einem den Brustkorb umschließenden Gurt, an deren Innenseite aufblasbare Polster angeordnet sind, um einen entsprechenden Druck auf den Brustkorb auszuüben.

Eine weitere Stützvorrichtung für den Brustkorb ist in FR-2670668 beschrieben.

Eine Stabilisierung des Brustkorbes ist nicht nur nach Rippenbrüchen erforderlich, sondern auch nach Brustöffnungen, wie sie beispielsweise im Zuge von Herzoperationen vorgenommen werden müssen. Nach Verschließen des Brustkorbes muss die entsprechende Wunde stabilisiert werden, um eine rasche Verheilung zu gewährleisten, wobei die Stabilisierung jedoch durch die beim Atmen auftretende Dehnung des Brustkorbes erschwert wird. Insbesondere beim Husten und bei anderen ruckartigen Ausdehnungen des Brustkorbes ist die Wunde erhöhten Beanspruchungen ausgesetzt und es zielt die vorliegende Erfindung daher darauf ab, eine Stützvorrichtung der eingangs genannten Art bereit zu stellen, durch welche der Brustkorb im Bereich der Wunde zur Beschleunigung der Heilung ausreichend stabilisiert wird und gleichzeitig jedoch die durch das Atmen hervorgerufene Ausdehnung des Brustkorbes unter Schonung des Wundbereichs zugelassen wird.

Zur Lösung dieser Aufgabe sind bei der erfindungsgemäßen Stützvorrichtung gemäß Anspruch 1 die Auflageelemehte von wenigstens zwei beiderseits des zu stützenden Brustkorbteils angeordneten Polstern gebildet und das Zugelement aufweist wenigstens zwei Abschnitte unterschiedlicher Elastizität, wobei ein erster, sich zwischen den beiden Polstern über den zu stützenden Brustkorbbereich erstreckender' Abschnitt in Zugrichtung eine geringere Elastizität aufweist als ein zweiter, sich zwischen den Polstern in entgegengesetzter Richtung erstreckender Abschnitt. Dadurch, dass die Auflageelemente von wenigstens zwei beiderseits des zu stützenden Brustkorbteils angeordneten Polstern gebildet sind, wird zum einen verhindert, dass das Zug- bzw. Spannelement unmittelbar auf dem zu stützenden Brustkorbteil und insbesondere der durch Öffnung des Brustkorbes entstandenen Wunde aufliegt, und zum anderen die Voraussetzung dafür geschaffen, dass der zwischen den Polstern liegende Bereich des Brustkorbes von Dehnungen im Wesentlichen freigehalten wird. Zu diesem Zweck ist nämlich erfindungsgemäß weiters vorgesehen, dass das Zugelement in dem sich zwischen den beiden Polstern über den zu stützenden Brustkorbbereich erstreckenden Abschnitt eine geringere Elastizität aufweist als der übrige Abschnitt des Zugelements. Die geringere Elastizität des Zugelements in dem sich über den zu stützenden Brustkorbbereich erstreckenden Abschnitt bzw. die Tatsache, dass das Zugelement in diesem Abschnitt bevorzugt im Wesentlichen überhaupt keine Elastizität aufweist, führt dazu, dass der Abstand zwischen den beiden die Wunde begrenzenden Polstern im Wesentlichen konstant gehalten wird, sodass in diesem sensiblen Bereich die Thoraxdehnung nicht zur Wirkung gelangt. Die Thoraxdehnung kommt vielmehr lediglich in dem sich zwischen den Polstern in entgegengesetzter Richtung erstreckenden Abschnitt zur Wirkung und somit lediglich in demjenigen Bereich, in welchem der Brustkorb intakt ist und somit keine Wundheilung erforderlich ist. Dadurch wird die Thoraxöffnungsoperationsnaht im postoperativen Bereich, d.h. z.B. nach Herz- oder Lungenoperationen, geschützt und es wird der Gefahr des Aufplatzens der Naht entgegengewirkt und damit der postoperativ auftretende Schmerz gelindert.

Zur Anpassung der erfindungsgemäßen Stützvorrichtung an die jeweiligen Bedürfnisse ist die Ausbildung bevorzugt derart weitergebildet, dass die Länge des ersten Abschnitts der Zugelementes verstellbar ist. Dadurch kann die wirksame Länge des im Bereich des zu stützenden Brustkorbteils als Abstandshalter zwischen den beiden Polstern wirksamen Zug- bzw. Spannelements eingestellt werden und gleichzeitig der insgesamt auf den Thorax wirkende Druck angepasst werden.

Um einen konstanten Abstand zwischen den beiden Polstern zu gewährleisten muss naturgemäß dafür Sorge getragen werden, dass die Polster ihre Lage relativ zum Brustkorb beibehalten und nicht verrutschen, da ein Verrutschen der Polster unmittelbar zu einer Belastung und damit zu einer Destabilisierung des Wundbereichs führen würde. Zum einen muss in diesem Zusammenhang dafür Sorge getragen werden, dass die Polster entsprechend rutschfest ausgebildet sind und es ist daher gemäß einer bevorzugten Weiterbildung der erfindungsgemäßen Stützvorrichtung vorgesehen, dass die Polster an der dem Brustkorb zugewandten Seite eine reibungserhöhende Schicht aufweisen. Zum anderen muss für einen ausreichenden Druck der Polster gegen den Brustkorb in radialer Richtung gesorgt werden, was bei der erfindungsgemäßen Konstruktion bereits dadurch begünstigt wird, dass das den Brustkorb umschließende Zug- bzw. Spannelement unter Zwischenschaltung des Polsters auf den Brustkorb zur Wirkung gelangt. Das Zug- bzw. Spannelement liegt im zu stützenden Bereich somit nicht unmittelbar auf dem Brustkorb auf und es wird dadurch die in Umfangsrichtung durch das Zug- bzw. Spannelement hervorgerufene Kraft im Bereich der Polster wirkungsvoll in eine radial wirkende Komponente zerlegt. Je höher der Polster ist, d.h. je größer der Abstand des Zugelements vom Brustkorb im Bereich der Polster ist, desto höher ist die von dem Polster auf den Brustkorb zur Wirkung gelangende Kraft.

Wie bereits erwähnt, wird die beim Atmen auftretende Thoraxdehnung von der erfindungsgemäßen Stützvorrichtung insoweit zugelassen als es die Elastizität des Zug- bzw. Spannelements zulässt, wobei die Elastizität vorwiegend in demjenigen Bereich des Zug- bzw. Spannelements vorgesehen ist, welcher außerhalb des zu stützenden Brustkorbbereichs liegt. Dieser zweite Abschnitt des Zug- bzw. Spannelements muss jedoch nicht über die gesamte Länge elastisch ausgebildet sein und es ist bevorzugt vorgesehen, dass der zweite Abschnitt in ihrer Elastizität begrenzte Dehnungszonen aufweist und außerhalb dieser Dehnungszonen im Wesentlichen keine Elastizität aufweist. Durch eine derartige Ausbildung wird es möglich die zulässige Ausdehnung des Zug- bzw. Spannelements zu begrenzen und zu kontrollieren, da bei Überschreiten der durch die Dehnungszonen freigegebenen Dehnung eine weitere Dehnung verhindert wird. Auf diese Art und Weise wird die Stützwirkung verbessert und der Halt der Stützvorrichtung erhöht.

Zur Anpassung der Stützvorrichtung an den jeweiligen Brustkorbumfang ist die Ausbildung bevorzugt derart weitergebildet, dass die Länge des zweiten Abschnitts verstellbar ist.

Bevorzugt ist das Zugelement von wenigstens zwei in Abstand voneinander den Brustkorb jeweils umschließenden Bändern bzw. Gurten gebildet. Durch das Vorsehen einer Mehrzahl von Bändern bzw. Gurten kann eine bessere Anpassung an die jeweilige Körperform vorgenommen werden und es können beispielsweise für die einzelnen Bänder bzw. Gurte unterschiedliche Längeneinstellungen getroffen werden. Auch die Breite der einzelnen Bänder bzw. Gurte kann von einander unterschiedlich getroffen werden, um eine optimale Druckverteilung und damit einen größeren Tragekomfort zu erreichen. Beispielsweise kann ein breiteres Band verwendet werden, wenn das betreffende Band höhere Zugkräfte aufbringen muss, um eine im Vergleich zu anderen Bändern gleichmäßige Flächenpressung zu erreichen. Der zwischen den einzelnen Bändern bzw. Gurten verbleibende Freiraum dient der besseren Luftzirkulation und vermindert lokale Aufwärmungen der Haut.

Zur weiteren Erhöhung des Tragekomforts kann bevorzugt vorgesehen sein, dass der zweite Abschnitt des Zug- bzw. Spannelements zusätzliche Polster trägt, welche zwischen dem Zugelement und dem Brustkorb zu liegen kommen, wobei die Anordnung der zusätzlichen Polster bevorzugt derart getroffen ist, dass die weiteren Polster im Seitenbereich des Brustkorbs zu liegen kommen. Dadurch wird eine Druckverteilung auf eine größere Fläche erreicht und es werden daher Einschnürungen der Haut bzw. einer allfälligen Fettschicht durch die einzelnen Gurte verhindert. Zusätzliche Polster können aber auch im Rückenbereich angeordnet sein, um den Komfort zu erhöhen.

Um die Variabilität der Stützeinrichtung zu erhöhen, ist bevorzugt vorgesehen, dass die Polster lösbar mit dem Zugelement verbunden sind. Die lösbare Verbindung kann in konventioneller Weise z.B. mit Hilfe von Klettverschlüssen erreicht werden und stellt die einfache Austauschbarkeit beispielsweise für Reinigungszwecke sicher. Außerdem wird die Möglichkeit geschaffen, zwischen verschiedenen Polsterungen auszuwählen.

Um den Halt der Stützvorrichtung zu verbessern, kann bevorzugt vorgesehen sein, dass das Zugelement mit zwei Schulterträgern verbunden ist. Derartige Schulterträger verhindern, dass die Stützvorrichtung am Brustkorb des Patienten abrutscht. Außerdem bewirkt ein Spannen der Schulterträger gleichzeitig auch eine Erhöhung des Zugs in Umfangsrichtung, das heißt eine Erhöhung des Drucks auf den Brustkorb.

Schließlich kann das Zugelement und/oder der Rückenteil mit Taschen zur Aufnahme von therapeutischen Geräten, wie z.B. Magneten oder elektrisch stimulierenden Elektroden, versehen sein. Derartige Taschen können aber beispielsweise auch für Kühlelemente verwendet werden, wenn dies in einzelnen Fällen, beispielsweise zur Schmerzlinderung, gewünscht ist.

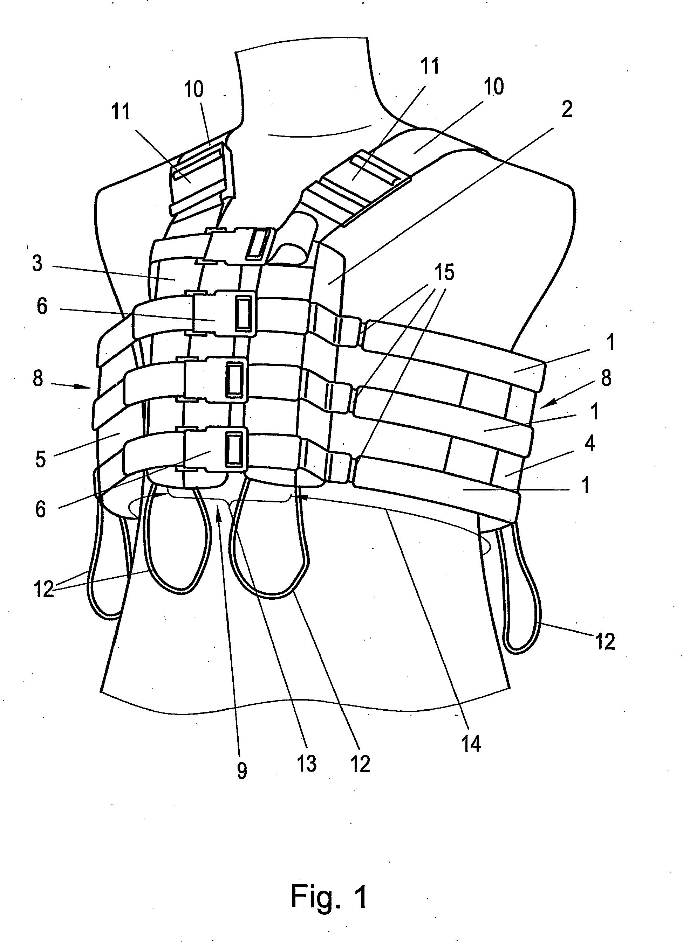

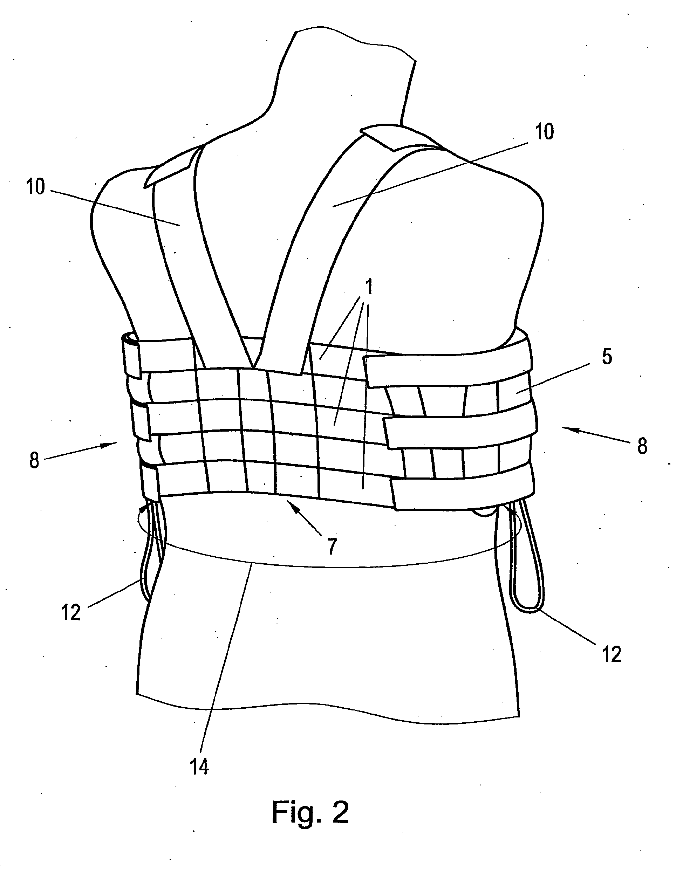

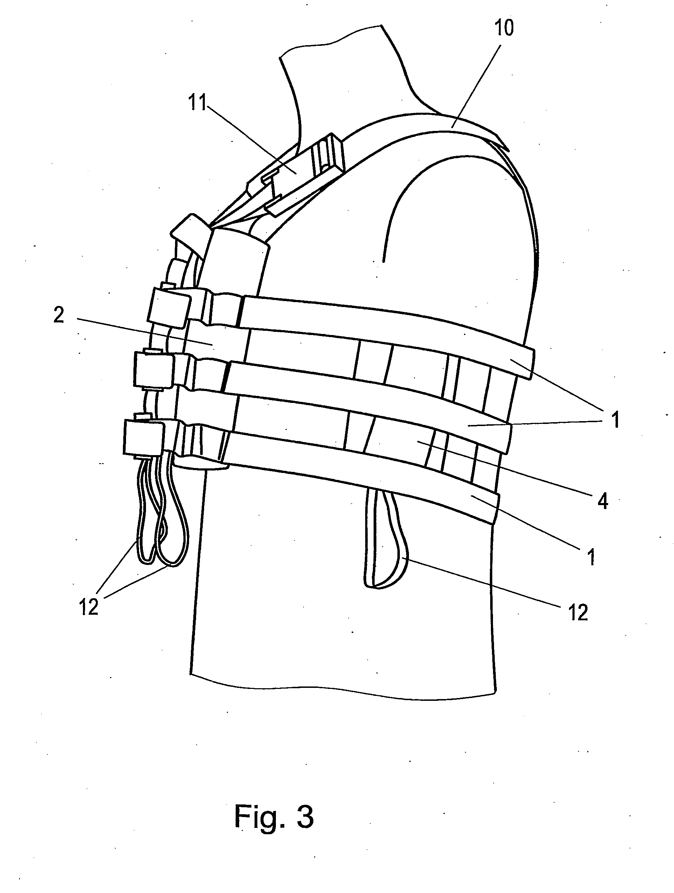

Die Erfindung wird nachfolgend anhand eines in der Zeichnung schematisch dargestellten Ausführungsbeispieles näher erläutert. In dieser zeigen Fig.1 eine Vorderansicht einer auf einer Puppe angebrachten erfindungsgemäßen Stützvorrichtung, Fig.2 eine Ansicht von hinten und Fig.3 eine Seitenansicht.

In den Figuren ist ersichtlich, dass die erfindungsgemäße Stützvorrichtung im Wesentlichen aus einem den Brustkorb umschließenden Zug- bzw. Spannelement sowie aus Polstern besteht, welche von dem Zugelement gegen den Brustkorb gedrückt werden. Das Zug- bzw. Spannelement besteht im vorliegenden Fall aus drei in Abstand voneinander jeweils den Brustkorb umschließenden Bändern (Zugelementen) 1, welche mit den Polstern 2,3,4 und 5 verbunden sind bzw. an diesen angreifen. Zwischen den Polstern 2 und 3 liegt hierbei der zu stützende Bereich des Brustkorbs, und zwar im vorliegenden Fall der Bereich des Brustbeins, wo der Brustkorb geöffnet wurde, um eine Herz- oder Lungenoperation durchzuführen. Die Polster 2 und 3 können aus konventionellem Material bestehen und sind mit den Bändern (Zugelementen) 1 bzw. dem Vorderteil 9 beispielsweise unter Verwendung eines Klettverschlusses verbunden, um eine leichte Austauschbarkeit zu gewährleisten. An der dem Brustkorb zugewandten Seite der Polster kann eine reibungserhöhende Schicht vorgesehen sein, welche beispielsweise aus Neopren besteht, sodass die Polster auf der Haut nicht verrutschen. Die Polster 2 und 3 können auch zur Ausbildung eines einzigen Polsterteils miteinander verbunden sein. Ein derartiger Polsterteil liegt beiderseits des zu stützenden Brustkorbbereichs am Brustkorb auf und weist in dem zu stützenden Brustkorbbereich eine Ausnehmung auf, und kann daher beispielsweise einen U-förmigen Querschnitt aufweisen.

Die den Brustkorb umschließenden Zugelemente 1 können in Umfangsrichtung jeweils einstückig ausgebildet sein, wobei zur leichteren Abnahme sowie dem leichteren Anziehen im mittleren Bereich Schnallen 6 vorgesehen sind. Im vorliegenden Fall ist jedoch keine einstückige Ausführung der Zugbänder 1 vorgesehen, sondern es ist ein Rückenteil 7 angeordnet, welcher zu beiden Seiten jeweils beispielsweise mit Hilfe von Klettverschlüssen mit einem Seitenteil 8 verbunden ist. Die Seitenteile 8 wiederum erstrecken sich bis zu den Polstern 2 bzw. 3, wobei die beiden Seitenteile 8 durch den Vorderteil 9 miteinander verbunden sind. Zwischen dem Vorderteil 9 und dem Rückenteil 7 erstrecken sich zwei Schulterträger 10, welche wiederum mit Hilfe von Schnallen 11 aufmachbar und in ihrer Länge verstellbar sind. Die Träger 10 verhindern ein Abrutschen der Stützvorrichtung. Sollte die Stützvorrichtung hingegen nach oben verrutschen, sind Schlaufen 12 vorne und/oder seitlich vorgesehen, mit welchen die Stützvorrichtung händisch nach unten gezogen werden kann.

Das von den Zugbändern 1 gebildete Zugelement weist einen ersten Abschnitt 13 auf, welcher sich zwischen den beiden Polstern 2 und 3 erstreckt. In diesem ersten Abschnitt 13 sind die Zugbänder 1 im Wesentlichen unelastisch, sodass zwischen den Polstern 2 und 3 ein gleich bleibender Abstand eingehalten wird. Vorausgesetzt, dass die Polster 2 und 3 relativ zum Brustkorb nicht verrutschen, hat dies zur Folge, dass der sich zwischen den Polstern 2 und 3 befindliche Brustkorbbereich sowie die durch die Brustkorböffnung entstandene Wunde stabilisiert werden. Die Gefahr einer Destabilisierung besteht nämlich während der durch das Atmen hervorgerufenen Thoraxdehnung und insbesondere bei ruckartigen Thoraxdehnungen, wie beispielsweise beim Husten. Durch die beiden Polster 2 und 3, welche stets in gleichem Abstand voneinander gehalten werden, wird wirksam verhindert, dass die Thoraxdehnung im Bereich der Wunde auftritt und es erfolgt die Thoraxdehnung vielmehr lediglich in dem verbleibenden Bereich des Brustkorbs außerhalb des durch die beiden Polster 2 und 3 begrenzten Bereichs. In diesem zweiten Abschnitt 14 der Zugbänder 1, nämlich im restlichen Umfangsabschnitt, welcher sich zwischen den Polstern 2 und 3 in entgegengesetzter Richtung erstreckt, bewirkt die Thoraxdehnung im Rahmen der Elastizität der Zugbänder 1 eine Längenveränderung der selben. Die Zugbänder 1 weisen zu diesem Zweck Dehnungszonen 15 auf, welche in ihrer Elastizität begrenzt sind. Die Dehnungszonen 15 bestehen hierbei aus einem elastischen Bandabschnitt sowie parallel dazu aus einem dehnungsfreien Bandabschnitt, welcher erst nach Übersteigen einer vorgegebenen Ausdehnung des elastischen Abschnitts zur Wirkung gelangt und eine weitere Ausdehnung verhindert.

Im vorliegenden Ausführungsbeispiel sind lediglich quaderförmige Polster gezeigt. Die Polster können naturgemäß aber auch abgeschrägt sein oder eine andere Form aufweisen und in ihrer Länge, Breite und Dicke unterschiedliche Dimensionen aufweisen. In die Polster können auch Kühlelemente integriert sein.

Um eine komfortable Anwendung auch bei Frauen zu ermöglichen, kann das mittlere der drei Zugelemente 1 weggelassen werden und es kann an dieser Stelle beispielsweise ein Büstenhalter in die Stützvorrichtung integriert werden. Das für die Zugbänder 1 verwendete Material kann aus Stoff oder auch aus Kunststoff bestehen.

## Patentansprüche

1. Stützvorrichtung für den Brustkorb mit wenigstens einem den Brustkorb umschließenden Zugelement (1) und Auflageelementen (2,3), die mit Hilfe des wenigstens einen Zugelements (1) gegen den Brustkorb andrückbar sind, wobei die Auflageelemente (2,3) von wenigstens zwei beiderseits des zu stützenden Brustkorbteils angeordneten Polstern (2,3) gebildet sind und dass das Zugelement (1) wenigstens zwei Abschnitte unterschiedlicher Elastizität aufweist, **dadurch gekennzeichnet, dass** ein erster, sich zwischen den beiden Polstern (2,3) über den zu stützenden Brustkorbbereich erstreckender Abschnitt (13) in Zugrichtung eine geringere Elastizität aufweist als ein zweiter, sich zwischen den Polstern (2,3) in entgegengesetzter Richtung erstreckender Abschnitt (14).

2. Stützvorrichtung nach Anspruch 1, **dadurch gekennzeichnet, dass** der erste, sich zwischen den beiden Polstern (2,3) über den zu stützenden Brustkorbbereich erstreckende Abschnitt (13) des Zugelements (1) in Zugrichtung keine Elastizität aufweist.

3. Stützvorrichtung nach Anspruch 1 oder 2, **dadurch gekennzeichnet, dass** die Länge des ersten Abschnitts (13) verstellbar ist.

4. Stützvorrichtung nach Anspruch 1, 2 oder 3, **dadurch gekennzeichnet, dass** der zweite Abschnitt (14) in ihrer Elastizität begrenzte Dehnungszonen (15) aufweist und außerhalb dieser Dehnungszonen (15) im Wesentlichen keine Elastizität aufweist.

5. Stützvorrichtung nach einem der Ansprüche 1 bis 4, **dadurch gekennzeichnet, dass** die Länge des zweiten Abschnitts (14) verstellbar ist.

6. Stützvorrichtung nach einem der Ansprüche 1 bis 5, **dadurch gekennzeichnet, dass** die Polster (2,3) an der dem Brustkorb zugewandten Seite eine reibungserhöhende Schicht aufweisen.

7. Stützvorrichtung nach einem der Ansprüche 1 bis 6, **dadurch gekennzeichnet, dass** das Zugelement (1) von wenigstens zwei in Abstand voneinander den Brustkorb jeweils umschließenden Zugbändern gebildet wird.

8. Stützvorrichtung nach einem der Ansprüche 1 bis 7, **dadurch gekennzeichnet, dass** der zweite Abschnitt (14) des Zugelements (1) zusätzliche Polster (4,5) trägt, welche bevorzugt in den Seitenbereichen und/oder im Rückenbereich des Brustkorbs zu liegen kommen.

9. Stützvorrichtung nach einem der Ansprüche 1 bis 8, **dadurch gekennzeichnet, dass** die Polster (2,3,4,5) lösbar mit dem Zugelement (16) verbunden sind.

10. Stützvorrichtung nach einem der Ansprüche 1 bis 9, **dadurch gekennzeichnet, dass** das Zugelement (1) mit zwei Schulterträgern (10) verbunden ist.

11. Stützvorrichtung nach einem der Ansprüche 1 bis 10, **dadurch gekennzeichnet, dass** das Zugelement (1) mit Taschen zur Aufnahme von therapeutischen Geräten, wie z.B. Magneten oder elektrisch stimulierenden Elektroden, versehen ist.

## Claims

1. A thorax supporting device including at least one tension element (1) surrounding the thorax and support elements (2,3) capable of being pressed against the thorax by the aid of the at least one tension element (1), wherein the support elements (2,3) are formed by at least two cushions (2, 3), which are arranged on both sides of the thoracic part to be supported, and that the tension element (1) comprises at least two portions of different elasticity, **characterized in that** a fist portion (13) extending between the two cushions (2, 3) over the thoracic region to be supported has a lower elasticity in the tension direction than a second portion (14) extending between the cushions (2, 3) in the opposite direction.

2. A supporting device according to claim 1, **characterized in that** the fist portion (13) of the tension element (1), which extends between the two cushions (2, 3) over the thoracic region to be supported, has no elasticity in the tension direction.

3. A supporting device according to claim 1 or 2, **characterized in that** the length of the first portion (13) is adjustable.

4. A supporting device according to claim 1, 2 or 3, **characterized in that** the second portion (14) comprises expansion zones (15) limited in elasticity and has substantially no elasticity outside said expansion zones (15).

5. A supporting device according to any one of claims 1 to 4, **characterized in that** the length of the second portion (14) is adjustable.

6. A supporting device according to any one of claims 1 to 5, **characterized in that the cushions (2, 3) are each provided** with a friction-enhancing layer on the side facing the thorax.

7. A supporting device according to any one of claims 1 to 6, **characterized in that** the tension element (1) is comprised of at least two tension straps each surrounding the thorax in a mutually spaced-apart relationship.

8. A supporting device according to any one of claims 1 to 7, **characterized in that** the second portion (14) of the tension element (1) carries additional cushions (4, 5) preferably coming to lie in the side regions and/or in the back region of the thorax.

9. A supporting device according to any one of claims 1 to 8, **characterized in that** the cushions (2, 3, 4, 5) are detachably connected with the tension element (1).

10. A supporting device according to any one of claims 1 to 9, **characterized in that** the tension element (1) is connected with two shoulder straps (10).

11. A supporting device according to any one of claims 1 to 10, **characterized in that** the tension element (1) is provided with pockets for receiving therapeutic aids such as, e.g. magnets or electrically stimulating electrodes.

## Revendications

1. Dispositif de soutien de la cage thoracique, comportant au moins un élément de traction (1) entourant la cage thoracique et des éléments d'appui (2, 3) qui peuvent être serrés contre la cage thoracique à l'aide dudit élément de traction (1) prévu au moins, dans lequel les éléments d'appui (2, 3) sont formés d'au moins deux coussins (2, 3) disposés de part et d'autre de la partie de cage thoracique à soutenir, et dans lequel l'élément de traction (1) présente au moins deux segments d'élasticité différente, **caractérisé en ce qu'**un segment (13), qui s'étend entre les deux coussins (2, 3) sur la zone de cage thoracique à soutenir, présente dans le sens de traction une élasticité inférieure à un second segment (14), qui s'étend entre les coussins (2, 3) dans le sens opposé.

2. Dispositif de soutien selon la revendication 1, **caractérisé en ce que** le premier segment (13) de l'élément de traction (1), qui s'étend entre les deux coussins (2, 3) sur la zone de cage thoracique à soutenir, ne présente aucune élasticité dans le sens de traction.

3. Dispositif de soutien selon la revendication 1 ou 2, **caractérisé en ce que** la longueur du premier segment (13) est réglable.

4. Dispositif de soutien selon la revendication 1, 2 ou 3, **caractérisé en ce que** le second segment (14) présente des zones d'allongement (15) d'élasticité limitée et ne présente essentiellement aucune élasticité en-dehors de ces zones d'allongement (15).

5. Dispositif de soutien selon l'une des revendications 1 à 4, **caractérisé en ce que** la longueur du second segment (14) est réglable.

6. Dispositif de soutien selon l'une des revendications 1 à 5, **caractérisé en ce que** les coussins (2, 3) présentent sur le côté orienté vers la cage thoracique une couche qui augmente la friction.

7. Dispositif de soutien selon l'une des revendications 1 à 6, **caractérisé en ce que** l'élément de traction (1) est formé d'au moins deux bandes de traction à distance l'une de l'autre et entourant respectivement la cage thoracique.

8. Dispositif de soutien selon l'une des revendications 1 à 7, **caractérisé en ce que** le second segment (14) de l'élément de traction (1) porte des coussins (4, 5) supplémentaires qui viennent de préférence reposer dans les zones latérales et/ou dans la zone arrière de la cage thoracique.

9. Dispositif de soutien selon l'une des revendications 1 à 8, **caractérisé en ce que** les coussins (2, 3, 4, 5) sont reliés à l'élément de traction (1) de façon amovible.

10. Dispositif de soutien selon l'une des revendications 1 à 9, **caractérisé en ce que** l'élément de traction (1) est relié à deux bretelles (10).

11. Dispositif de soutien selon l'une des revendications 1 à 10, **caractérisé en ce que** l'élément de traction (1) est muni de poches permettant de loger des appareils thérapeutiques, comme par exemple des aimants ou des électrodes de stimulation électrique.
